(19) Europäisches Patentamt
European Patent Office
Office européen des brevets

(11) Numéro de publication : **0 283 362 B1**

(12) # FASCICULE DE BREVET EUROPEEN

(45) Date de publication du fascicule du brevet : **17.07.91 Bulletin 91/29**

(51) Int. Cl.[5] : **C07C 315/02**, C07C 317/00, A61K 31/16

(21) Numéro de dépôt : **88400432.6**

(22) Date de dépôt : **24.02.88**

(54) **2-(benzhydrylsulfonyl) acétamide, son procédé de préparation et son application en thérapeutique.**

(30) Priorité : **26.02.87 FR 8702586**

(43) Date de publication de la demande : **21.09.88 Bulletin 88/38**

(45) Mention de la délivrance du brevet : **17.07.91 Bulletin 91/29**

(84) Etats contractants désignés : **AT BE CH DE ES FR GB GR IT LI LU NL SE**

(56) Documents cités : **FR-A- 2 385 693**

(73) Titulaire : **LABORATOIRE L. LAFON
19 Avenue du Professeur Cadiot
F-94701 Maisons Alfort (FR)**

(72) Inventeur : **Lafon, Louis
5 rue de l'Alboni
F-75016 Paris (FR)**

(74) Mandataire : **Le Guen, Gérard et al
CABINET LAVOIX 2, place d'Estienne d'Orves
F-75441 Paris Cédex 09 (FR)**

**EP 0 283 362 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne le 2-(benzhydrylsulfonyl)acétamide, son procédé de préparation et son application en thérapeutique.

La présente invention a ainsi pour objet le 2-(benzhydrylsulfonyl)acétamide qui est un composé de formule.

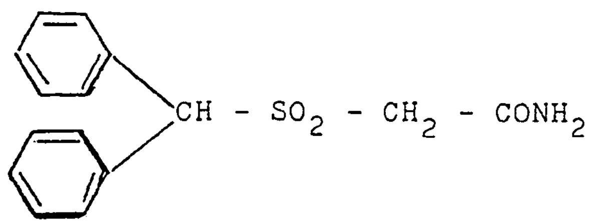

I

La demanderesse a découvert que ce composé présente une activité sur le système nerveux central et peut être utilisé en thérapeutique.

Le 2-(benzhydrylsulfonyl)acétamide peut être préparé par oxydation, notamment par l'eau oxygénée, du 2-(benzhydrylthio)acétamide, lui même préparé par réaction de l'ammoniaque sur le chlorure de l'acide 2-(benzhydrylthio)acétique.

La présente invention a également pour objet des compositions thérapeutiques comprenant à titre de principe actif le 2-(benzhydrylsulfonyl)acétamide.

L'exemple suivant illustre la préparation du composé :

a) Préparation du 2-(benzhydrylthio)acétamide

21 g (0,076 mole) de chlorure de benzydrylthioacétyle, en solution dans 100 ml de chlorure de méthylène, sont ajoutés goutte à goutte en agitant dans 40 ml d'ammoniaque et 40 ml d'eau. Après une heure d'agitation, la phase organique est décantée, lavée à l'eau et séchée sur $Na_2SO_4$. On évapore le solvant sous vide, cristallise le résidu dans l'éther isopropylique et recristallise dans l'acétate d'éthyle. On obtient le composé avec un rendement de 45%.

C'est une poudre blanche, soluble dans les alcools, l'acétone, l'acétate d'éthyle ; insoluble dans l'eau, l'éther isopropylique.

Il fond à 107-108°C.

b) Préparation du 2-(benzhydrylsulfonyl)acétamide (n° de code CRL 41 056)

12,85 g (0,05 mole) de 2-(benzhydrylthio)acétamide en solution dans 50 ml d'acide acétique sont oxydés par addition goutte à goutte entre 20 et 30°C de 15 ml (0,15 mole) d'eau oxygénée à 110 volumes. Après 5 heures d'agitation, le mélange est laissé en contact 48 heures. La sulfone est essorée, lavée à l'eau, séchée et recristallisée dans l'éthanol.

On obtient le composé avec un rendement globale de 36%.

Il se présente sous la forme d'aiguilles blanches, soluble dans les alcools, l'acétone ; peu soluble dans le chloroforme, l'acétate d'éthyle ; insoluble dans l'éther éthylique, l'eau.

Il fond à 194°C.

On donnera ci-après des résultats des études pharmacologiques du composé (CRL 41 056) mettant en évidence les propriétés du composé.

Le CRL 41 056, en suspension dans une solution de gomme arabique, a été administré par voie intrapéritonéale sous un volume de 20 ml/kg chez la souris (mâle, NMRI, Evic Ceba) et 5 ml/kg chez le rat (mâle, $CD_1$, SPRAGUE DAWLEY, Charles).

I - Pretoxicité (3 souris par dose).

— Aux doses de 64, 128 et 256 mg/kg apparition de crampes abdominales
— A la dose de 512 mg/kg : crampes abdominale, dyspnée
— A la dose de 1024 mg/kg : crampes abdominales, dyspnée, sédation, pas de mortalité.

II - Comportement global et reactivité

Des lots de 3 rats sont observés avant, puis 15 mn, 30 mn, 1 h, 2 h, 3 h et 24 h après l'administration de CRL 41 056.

— 4 mg/kg : hypo-réactivité au toucher (30 mn)
— 16 mg/KG : hypo-réactivité au toucher (30 mn)
: mydriase pendant 2 à 3 heures
— 64 mg : kg : hypo-réactivité au toucher (30 à 60 mn)
: hypotonie musculaire (15 mn)
: mydriase pendant 1 heure
—256 mg/kg : hypo-réactivité au toucher et hypotonie musculaire pendant 30 mn.
mydriase pendant 1 à 3 heures
dyspnée pendant 2 heures

III - Action sur la motilité spontanée

Une demi-heure après avoir reçu le CRL 41 056, les souris (6 par dose, 12 témoins) sont placées en actimètre où leur motilité est enregistrée pendant 30 minutes.

Aux doses de 128 et 512 mg/kg, le CRL 41 056 provoque une diminution de l'activité motrice spontanée.

Conclusion

Le CRL 41 056 montre un effet sédatif avec hypomotilité et hypo-réactivité.

Le CRL 41 056 s'est avéré être un bon sédatif chez l'homme à la dose de deux comprimés par jour dosés de 100 mg.

Les compositions thérapeutiques selon l'invention peuvent être administrées à l'homme ou aux animaux par voie orale et parentérale.

Elles peuvent être sous la forme de préparations solides ou semi-solides. Comme exemple, on peut citer les comprimés, les gélules les suppositoires, ainsi que les formes-retard.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

**Revendications**

**Revendications pour les Etats contractants : AT, BE, CH, DE, FR, GB, IT, LI, LU, NL, SE**

1. Le 2-(benzhydrylsulfonyl)acétamide.
2. Procédé de préparation du 2-(benzhydrylsulfonyl)acétamide, caractérisé en ce que l'on oxyde le 2-(benzhydrylthio)acétamide.
3. Composition thérapeutique comprenant à titre de principe actif un composé selon la revendication 1.

**Revendications pour l'Etat contractant suivant : ES**

1. Procédé de préparation du 2-(benzhydrylsulfonyl)acétamide, caractérisé en ce que l'on oxyde le 2-(benzhydrylthio)acétamide.
2. Procédé de préparation d'une composition thérapeutique caractérisé en ce que l'on met le 2-(benzhydrylsulfonyl)acétamide sous une forme pharmaceutiquement acceptable.

**Revendications pour l'Etat contractant suivant : GR**

1. Le 2-(benzhydrylsulfonyl)acétamide.
2. Procédé de préparation du 2-(benzhydrylsulfonyl)acétamide, caractérisé en ce que l'on oxyde le 2-(benzhydrylthio)acétamide.
3. Procédé de préparation d'une composition thérapeutique caractérisé en ce que l'on met un composé

selon la revendication 1, sous une forme pharmaceutiquement acceptable.

## Claims

### Claims for the Contracting States : AT, BE, CH, DE, FR, GB, IT, LI, LU, NL, SE

1. 2-(benzhydrylsulfonyl)acetamide.

2. Process for the preparation of 2-(benzhydrylsulfonyl)acetamide, characterised in that 2-(benzhydrylthio)-acetamide is oxidized.

3. Therapeutic composition comprising a compound according to claim 1 as active ingredient.

### Claims for the Contracting State : ES

1. Process for the preparation of 2-(benzhydrylsulfonyl)acetamide, characterised in that 2-(benzhydrylthio)-acetamide is oxidized.

2. Process for the preparation of a therapeutic composition, characterised in that 2-(benzhydrylsulfonyl)-acetamide is put into a pharmaceutically acceptable form.

### Claims for the Contracting State : GR

1. 2-(benzhydrylsulfonyl)acetamide.

2. Process for the preparation of 2-(benzhydrylsulfonyl)acetamide, characterised in that 2-(benzhydrylthio)-acetamide is oxidized.

3. Process for the preparation of a therapeutic composition, characterised in that a compound according to claim 1 is put into a pharmaceutically acceptable form.

## Patentansprüche

### Patentansprüche für die Vertragsstaaten : AT, BE, CH, DE, FR, GB, IT, LI, LU, NL, SE

1. 2-(Benzhydrylsulfonyl)-acetamid.

2. Verfahren zur Herstellung von 2-(Benzhydrylsulfonyl)-acetamid, **dadurch gekennzeichnet**, daß man 2-(Benzhydrylthio)-acetamid oxidiert.

3. Therapeutische Zubereitung enthaltend als Wirkstoff eine Verbindung nach Anspruch 1.

### Patentansprüche für den Vertragsstaat : ES

1. Verfahren zur Herstellung von 2-(Benzhydrylsulfonyl)-acetamid, **dadurch gekennzeichnet**, daß man 2-(Benzhydrylthio)-acetamid oxidiert.

2. Verfahren zur Herstellung einer therapeutischen Zubereitung, **dadurch gekennzeichnet**, daß man 2-(Benzhydrylsulfonyl)-acetamid in eine pharmazeutisch annehmbare Form bringt.

### Patentansprüche für den Vertragsstaat : GR

1. 2-(Benzhydrylsulfonyl)-acetamid.

2. Verfahren zur Herstellung von 2-(Benzhydrylsulfonyl)-acetamid, **dadurch gekennzeichnet**, daß man 2-(Benzhydrylthio)-acetamid oxidiert.

3. Verfahren zur Herstellung einer therapeutischen Zubereitung, **dadurch gekennzeichnet**, daß man eine Verbindung nach Anspruch 1 in eine pharmazeutisch annehmbare Form bringt.